# EUROPEAN PATENT SPECIFICATION

(11) **EP 4 098 188 B1**
(45) Date of publication and mention of the grant of the patent: **27.03.2024**
(21) Application number: 21177149.8
(22) Date of filing: 01.06.2021
(51) Int. Cl.: A61B 5/11, A61B 5/22

(54) **MONITORING KIT WITH PRESSURE MEASURING DEVICE AND COMPUTER-IMPLEMENTED EVALUATION PROGRAM**
MONITORINGKIT MIT DRUCKMESSVORRICHTUNG UND COMPUTERIMPLEMENTIERTEM BEURTEILUNGSPROGRAMM
KIT AVEC DISPOSITIF DE MESURE DE PRESSION ET PROGRAMME D'ÉVALUATION MIS EN OEUVRE PAR ORDINATEUR

(43) Date of publication of application: 07.12.2022
(73) Proprietor: medac Gesellschaft für klinische Spezialpräparate mbH, 22880 Wedel (DE)
(72) Inventor: Nammari, David, 22880 Wedel (DE); Wehner, Thomas, 22045 Hamburg (DE)
(74) Representative: Vossius & Partner Patentanwälte Rechtsanwälte mbB

(56) References cited:
- EP-A1- 2 540 226
- GB-A- 2 475 659
- US-A1- 2011 066 078
- US-A1- 2011 088 463
- US-A1- 2017 265 802
- US-A1- 2019 328 321

## Description

Arthritis is an inflammation of the joints and can affect one joint or multiple joints of a human body. There are many different types of arthritis. Two of the most common types are osteoarthritis (OA), often referred to as arthrosis, and rheumatoid arthritis (RA).

Cartilage is a firm but flexible connective tissue in the joints. It protects the joints by absorbing forces caused by movement and/or stressing of the joints. Arthritis, in particular OA, may be caused by normal wear and tear of such cartilage, which may lead to a reduction in the amount of the cartilage in the joint. This can cause joint pain, stiffness, and swelling in the joint.

RA, on the other hand, is an autoimmune and inflammatory disease. It occurs when a human body's immune system attacks the tissues of the body. RA primarily attacks the joints, usually many joints at once, and commonly affects joints in the hands, wrists, and knees. The affected joints may become inflamed due to RA, causing damage to joint tissue, which can cause long-lasting or chronic pain, lack of balance, and deformity.

Arthritis, such as OA and RA, are commonly treated with the help of drugs. However, research has shown that regular physical activity may decrease pain, improve function and delay disability caused by arthritis.

Moreover, monitoring the conditions of arthritis and/or arthrosis can provide valuable information regarding a deterioration or an improvement of the conditions of the arthritis or arthrosis, for instance due to a treatment involving movement therapy, such as physical therapy and/or ergotherapy and/or a drug treatment. Based on this information, the patient and/or a health practitioner can adjust the treatment of the arthritis or arthrosis accordingly. For instance, if it is determined that the condition of the arthritis or arthrosis of a patient is deteriorating despite the use of a certain form of treatment, such as a certain form of physical therapy and/or ergotherapy, the patient and/or a health practitioner may adjust the treatment accordingly and further monitor the conditions of the arthritis or arthrosis until an effective and/or optimal treatment may be determined.

Monitoring equipment according to the state of the art is for instance described in GB2475659A.

However, the above-identified issues have not or at least not sufficiently been addressed in the prior art. Thus, improvements are desired to improve the quality of life of patients who suffer from a form of arthritis or arthrosis.

It is therefore an object of the present invention to provide an improved means for monitoring a medical condition of a user related to arthritis and/or arthrosis.

This object is achieved by a kit for monitoring a medical condition of a user related to arthritis and/or arthrosis defined by the features of claim 1. Preferred variations and further developments are defined by the features of the dependent claims.

The pressure measuring device of the kit comprises an elastic body defining a lumen filled with a compressible fluid. The lumen is configured to be compressed by at least one body part of a user.

The elastic body may be deformed by a force exerted by the at least one body part, such as a hand, finger or foot, of the user onto at least a portion of the elastic body. By deforming the elastic body the lumen, and thus the compressible fluid disposed therein, may be compressed. Preferably, the elastic body may be made at least partially of a soft material, preferably of a soft synthetic or natural polymer material, such as silicone, polyethylene, polyurethane, soft PVC, (natural) rubber and/or latex, so that the elastic body can be gripped and compressed more easily by the user and discomfort to the body part of the user in contact with the elastic body can be avoided or at least reduced.

The pressure measuring device further comprises a pressure detection device arranged at least partially within the lumen. The pressure detection device may be arranged completely within the lumen, which may provide a better compressibility of the elastic body and the lumen and a more comfortable feel for the user when gripping and compressing the elastic body and the lumen since a wall of the elastic body surrounding the lumen may act as a cushion-like barrier between the body part of the user and the pressure detection device.

An additional securing means may be provided, preferably at least partially within the lumen, to secure at least the pressure detection device in the elastic body to prevent or at least limit movement of the pressure detection device relative to the elastic body.

However, the pressure detection device may also be arranged only partially within the lumen.

The portion of the pressure detection device not arranged in the lumen may, for instance, be arranged in a wall of the elastic body. In this case, a pressure detecting element of the pressure detection device, such as a piezoelectric element, a piezoresistive element or a deflectable membrane, which actually physically detects the pressure in the lumen via at least partial physical exposure to the fluid in the lumen, may be arranged in the lumen to ensure that the pressure detecting element is exposed to the fluid in the lumen.

Components of the pressure detection device which do not have to be exposed to the fluid within the lumen in order to detect the pressure of the fluid within the lumen by means of the pressure detection device, such as a circuit board or at least portions thereof, a power source, etc., may be arranged outside of the lumen, such as in a wall of the elastic body. Thus, the pressure detection device may be partially, e.g., certain components thereof, arranged in the lumen and partially, e.g., other components thereof, within a wall of the elastic body.

By arranging at least a portion of the pressure detection device in a wall of the elastic body, the pressure detection device may be fixed to the wall of the elastic body to prevent or at least limit movement of the pressure detection device relative to the elastic body.

The pressure detection device is configured to detect at least one absolute pressure in the lumen, generate a pressure signal based on the detected absolute pressure, and provide the pressure signal to an evaluating device for evaluating the pressure signal.

Preferably, a smoothing filter, preferably a median filter, is provided for smoothing the pressure signal. Preferably, the smoothing filter is a band pass filter or a low pass filter. However, other smoothing filter types are also feasible.

The pressure detection device may include a transmitting element configured to transmit the pressure signal to the evaluating device.

The pressure detecting element may be a micro pressure sensor, preferably a piezoresistive silicon pressure sensor.

Preferably, the pressure detection device is configured as a unitized device which includes all or at least a majority of the components required for the operation of the pressure detection device in order to detect the pressure of the fluid within the lumen. Preferably, such a unitized pressure detection device has maximum width x length x height dimensions of 20 mm x 20 mm x 16 mm. By providing a unitized pressure detection device with such compact maximum dimensions the pressure detection device can be more easily arranged in the pressure measuring device in a non-intrusive or at least minimally intrusive manner. Thus, no or at least less discomfort may be caused to the body part of the user in contact with the elastic body, in particular when compressing the elastic body and the lumen. Moreover, damage to the pressure detection device may be avoided or at least minimized when the elastic body and the lumen is compressed by the body part of the user by providing a small pressure detection device.

The pressure measuring device further comprises an energy source arranged within the elastic body, preferably at least partially within the lumen. The energy source is configured to provide electrical energy to at least the pressure detection device. The energy source may comprise at least one battery. Preferably, the energy source may comprise a plurality of batteries. Preferably, the battery is a rechargeable accumulator or the plurality of batteries is a plurality of rechargeable accumulator, respectively.

The energy source may be arranged completely within the lumen. This may provide a better compressibility of the elastic body and the lumen and a more comfortable feel for the user when gripping and compressing the elastic body and the lumen since a wall of the elastic body surrounding the lumen may act as a cushion-like barrier between the energy source and the body part of the user in this case.

In this case, an additional securing means is preferably provided, preferably at least partially within the lumen, to prevent or at least limit movement of the energy source relative to the elastic body.

The energy source may also be arranged partially within the lumen and partially within a wall of the elastic body. Alternatively, the energy source may be arranged completely in a wall of the elastic body. By arranging at least a portion of the energy source in a wall of the elastic body, the energy source may be fixed to the wall of the elastic body to prevent or at least limit movement of the energy source relative to the elastic body.

Thus, the pressure measuring device for monitoring a medical condition of a user related to arthritis and/or arthrosis according to the present invention aids the user or a health practitioner in assessing the force which can be exerted by a certain body part, for instance a hand, of the user by detecting the absolute pressure which is prevalent in the lumen when the user compresses the lumen via the elastic body, generating a pressure signal based on the detected absolute pressure and providing the pressure signal to an evaluating device for evaluating the pressure signal.

The evaluating device may process, and preferably also store, the signal and provide various information to the user based on the generated pressure signal, such as at least one of a development over a specific time, warnings when the detected absolute pressure exceeds or falls below predetermined thresholds and suggested treatments including movement therapy, such as physical therapy and/or ergotherapy and/or drug treatment. A suggested drug treatment may include, e.g., informing the user to take a specific drug or combination of drugs or a specific drug dose or drug doses. The information may alternatively or additionally include informing the user to seek a health professional.

The evaluating device may be an external component, such as a smartphone, a PC or a tablet, coupled, preferably wirelessly, for instance via a Bluetooth connection, to the pressure detection device. Third party components may be used as an evaluating device. Hence, the conditions of the arthritis or arthrosis of the user, in particular regarding a deterioration or an improvement of the conditions of the arthritis or arthrosis, can be closely monitored, for instance on a daily basis, in order to adapt and/or optimize the user's treatment.

The pressure measuring device may be used intuitively and easily by the user so that a health practitioner or technical expert does not have to be present to conduct the monitoring. Thus, the conditions of the arthritis or arthrosis of the user may be monitored in a daily environment of the user, for instance at the user's home or workplace. This may allow a more convenient and consistent monitoring of the conditions of the arthritis or arthrosis of the user, which may lead to a more effective monitoring to allow the treatments, including movement therapy, such as physical therapy and/or ergotherapy and/or drug treatment, to be adapted and/or adjusted more accurately to the needs of the user.

The pressure measuring device may be configured to be squeezed by a hand of the user and/or between the shank and the thigh of a leg of the user and/or by a foot of the user.

The pressure measuring device and/or the evaluating device is configured to compare the detected absolute pressure with at least one predetermined lower absolute pressure value and to trigger a warning signal if the detected absolute pressure is less than the predetermined lower absolute pressure value. Optionally, the pressure measuring device and/or the evaluating device may be configured to compare the detected absolute pressure with at least one predetermined upper absolute pressure value and to trigger a warning signal if the detected absolute pressure is greater than the predetermined upper absolute pressure value.

Such a configuration is to be understood as also encompassing the pressure measuring device and/or the evaluating device being configured to compare a value derived from the detected absolute pressure with at least one predetermined value which correlates with pressure, e.g., a force value.

For instance, the configuration described above also encompasses the pressure measuring device being configured to compare a force value derived from the detected absolute pressure with at least one predetermined upper force value and/or at least one predetermined lower force value and to trigger a warning signal if the derived force value is less than the predetermined lower force value and/or greater than the predetermined upper force value.

For this purpose, the pressure measuring device and/or the evaluating device may be configured to correlate a detected absolute pressure value with a corresponding force value.

By triggering a warning signal when the detected absolute pressure/force value is less than a predetermined lower absolute pressure value/force value, the user and/or a health practitioner may be informed that a critical strength threshold of the body part of the user, for instance a hand, has not been met, which may indicate that the conditions of the arthritis or arthrosis of the user has deteriorated and/or which may indicate a condition which is not beneficial and/or is harmful to the user. Thus, based on this warning, the treatment(s) for the conditions of the arthritis or arthrosis of the user, including movement therapy, such as physical therapy and/or ergotherapy and/or drug treatment, may be adapted and/or adjusted accordingly with or without the aid of a health professional to aim at improving the conditions.

By triggering a warning signal when the detected absolute pressure/force value is greater than a predetermined absolute pressure value/force value, the user and/or a health practitioner may be informed that a certain predetermined strength threshold has been surpassed, which may be less beneficial or even harmful to the user.

A variety of predetermined pressure values may be stored in the pressure measuring device and/or the evaluating device. For instance, different predetermined pressure values may be provided for monitoring the strength of different body parts of the user. The pressure measuring device and/or the evaluating device may provide a variety of different predetermined measuring options e.g., at different body parts of the user and/or with different measurement settings, which the user may select from. Accordingly, the appropriate predetermined absolute pressure values may thereby be determined.

For instance, the pressure measuring device and/or the evaluating device may offer a variety of different predetermined measuring situations including a hand test, in which the user may compress the elastic body and thus the lumen with an entire hand, a finger test, in which the user may compress the elastic body and thus the lumen with just one, two, three or four fingers, and a thumb test, in which the user may compress the elastic body and thus the lumen with just a thumb.

The predetermined absolute pressure values may also be dependent on different age groups, e.g., child, youth or adult, and/or on gender. Thus, the pressure measuring device and/or the evaluating device may offer a selection to the user to select the appropriate age group and/or gender. This may allow monitoring the conditions of the arthritis or arthrosis of the user more accurately according to the present situation of the user.

Preferably, the pressure detection device, and preferably also the energy source, may be at least partially embedded in a wall of the elastic body. This allows for an efficient means of securing the pressure detection device to the wall of the elastic body to prevent or at least limit movement of the pressure detection device relative to the elastic body without the need for additional securing means.

Alternatively or additionally, the pressure measuring device may further comprise a compressible sponge material and/or foam material and/or gel and/or wadding material arranged within and at least partially filling the lumen. The pressure detection device, and preferably also the energy source, may be arranged at least partially within the sponge material and/or foam material and/or gel and/or wadding material to secure the pressure detection device, and preferably also the energy source, in the pressure measuring device. Alternatively, other materials, preferably soft and/or deformable materials, may be arranged within the lumen to at least partially fill the lumen. For instance, a gel and/or a wadding may be used instead of or in addition to the sponge or foam material.

The sponge material and/or foam material and/or gel and/or wadding material may allow securing the pressure detection device within the sponge material and/or foam material and/or gel and/or wadding material to prevent or at least limit movement of the pressure detection device relative to the elastic body. In this case, securing the pressure detection device is not reliant on a wall of the elastic body. Thus, for instance, the pressure detection device can be arranged entirely within the lumen, preferably at a distance from the wall of the elastic body. Preferably, the pressure detection device and/or other components arranged within the lumen may be arranged substantially centrally in the lumen.

The wall of the elastic body and the sponge material and/or foam material and/or gel and/or wadding material may act as a cushion-like barrier between the body part of the user and the pressure detection device and/or other components arranged within the lumen to provide a better compressibility of the elastic body and the lumen and a more comfortable feel for the user when gripping and compressing the elastic body and the lumen. Thus, the pressure detection device and/or other components arranged within the lumen may be less intrusive to the user when compressing the elastic body and the lumen.

Preferably, the pressure measuring device may further comprise a temperature detection device arranged at least partially within the lumen. The temperature detection device may be configured to detect at least one temperature in the lumen. The detected temperature may be provided to the evaluating device.

The detected temperature may be used to correct the generated pressure signal based on a deviation of the detected temperature from a reference temperature, e.g., a reference temperature at which the pressure detection device may have been calibrated. Thus, drifts of the generated pressure signal based on different temperatures in the environment surrounding the pressure detection device, i.e., in the lumen, may be compensated for a more accurate pressure measurement.

The temperature detection device and the pressure detection device may be configured as a single, coherent unit. Alternatively, the temperature detection device and the pressure detection device may be configured as separate units.

Preferably, the elastic body may comprise two half-shells which are preferably fixedly connected, preferably adhesively, to each other in a substantially fluid-tight manner. Thus, the internal components of the pressure measuring device may be placed into one or both of the two half-shells, depending on their desired arrangement in the pressure measuring device. The two half-shells may then be fixedly connected, preferably adhesively, to each other in a fluid-tight manner. This may allow for a simple and quick assembly of the pressure measuring device.

Preferably, the pressure measuring device may further comprise at least one vibration device arranged at least partially within the pressure measuring device. The vibration device may be configured to vibrate upon receiving a vibration triggering signal. The vibration device may thus provide haptic signals to the user to indicate a certain event to the user based on the vibration triggering signal.

Preferably, the pressure measuring device and/or the evaluating device may be configured to generate the vibration triggering signal to activate the vibration device for at least one of the following events: upon the evaluating device being communicatively connected with at least the pressure detection device, and when the user compresses the lumen of the elastic body while the evaluating device is communicatively disconnected from the pressure detection device.

By indicating to the user when the evaluating device is communicatively connected with at least the pressure detection device via vibration, the user may be informed when the user may begin compressing the elastic body and the lumen at the start of the measurement.

By indicating to the user when the user compresses the lumen of the elastic body while the evaluating device is communicatively disconnected from the pressure detection device, the user may be informed that the user should stop compressing the elastic body and the lumen since no data can be transmitted to the evaluating device due to a lack of communicative connection between the pressure detection device and the evaluating device. This may enhance the user-friendliness and efficiency of the pressure measuring device.

Preferably, the energy source may be configured to be recharged, preferably wirelessly.

Preferably, the energy source may be at least one rechargeable battery and the pressure measuring device may comprise at least one, preferably magnetic, connection element electrically coupled to the battery and configured to be coupled to a power source outside of the pressure measuring device. The connection element may preferably be embedded at least partially in a wall of the elastic body. Preferably, the connection element extends completely through the wall of the elastic body. The connection element may provide a convenient, intuitive and efficient connection means for connecting the rechargeable battery to an outside power source to recharge the battery.

Configuring the connection element to be magnetic may provide a more secure connection between the power source and the connection element. Moreover, the process of connecting the power source to the connection element may thereby be facilitated.

By embedding the connection element at least partially in a wall of the elastic body, the connection element may be secured to prevent or at least limit movement of the connection element device relative to the elastic body without the need for additional securing means. Moreover, the connection element can be easily accessed from outside of the elastic body in this case.

Preferably, the pressure measuring device may further comprise at least one accelerometer or gyroscope arranged at least partially within the pressure measuring device. The accelerometer may be configured to detect movements, such as shaking, of the pressure measuring device.

Alternatively or additionally, a gyroscope configured to detect movements, such as shaking, of the pressure measuring device may be provided at least partially within the pressure measuring device.

Preferably, the accelerometer may be configured to detect a tremor of the user and generate a tremor signal. The tremor signal may be provided, e.g., via a transmitting device, preferably a wireless transmitting device configured to wirelessly communicate with the evaluating device, to the evaluating device.

Tremors in body parts, such as the hands, of a user, can indicate at least one particular condition of the user, the condition preferably not being beneficial and/or being harmful to the user. Thus, detecting such tremors, generating a tremor signal and providing the tremor signal to the evaluating device by means of the accelerometer may be helpful in detecting such a condition, in particular an overloaded or overworked condition. Thus, by providing an alert in such a condition the user may be warned and can thus avoid such a condition, such as an overloaded or overworked condition.

Alternatively or additionally, a gyroscope may be provided and configured to detect a tremor of the user and generate a tremor signal and provide the tremor signal to the evaluating device.

Preferably, the pressure measuring device may be configured to determine the frequency and/or the amplitude of the detected tremor based on the tremor signal and trigger a warning signal if: the determined frequency surpasses a predetermined frequency and/or the determined amplitude surpasses a predetermined amplitude, and/or the determined frequency falls within a predetermined frequency range and/or the determined amplitude falls within a predetermined amplitude range.

Preferably, the tremor signal may first be filtered, e.g., via a smoothing filter, preferably a median filter. Preferably, the smoothing filter is a band pass filter or a low pass filter. However, other smoothing filter types are also feasible.

Preferably, the determined amplitude may then be compared to the predetermined amplitude and/or the predetermined amplitude range. If the determined amplitude surpasses the predetermined amplitude and/or falls within the predetermined amplitude range, then preferably the determined frequency may then be compared to the predetermined frequency and/or the predetermined frequency range to assess whether a warning signal should be triggered.

Preferably, the pressure measuring device may be configured to additionally consider at least one value of the detected absolute pressure and/or at least one force value derived therefrom when assessing whether a warning signal should be triggered.

The pressure measuring device may be configured to compare the detected absolute pressure/force value with at least one predetermined absolute pressure/force value and trigger a warning signal if the detected absolute pressure/force value is lower and/or greater than a predetermined absolute pressure/force value.

Preferably, the predetermined frequency range may be from 4 Hz to 13 Hz since physiological tremors in humans commonly lie in this range.

Preferably, the accelerometer may have a sampling frequency of at least 30 Hz, preferably at least 40 Hz, more preferably at least 50 Hz, most preferably at least 60 Hz. This may allow for the frequency of the tremor to be accurately determined so that the warning signal may be triggered for tremors having a frequency which lies in the predetermined frequency range.

Preferably, the pressure measuring device may further comprise a transmitting device arranged within the elastic body and configured to transmit the signals generated by the pressure measuring device to the evaluating device. The transmitting device may be fixed, directly or indirectly, to at least the pressure detection device. The transmitting device may be a Bluetooth transmitter. Alternatively, the transmitting device may be any other wirelessly transmitting device.

The present invention relates to a kit comprising an evaluating device and the pressure measuring device according to claim 1. While the evaluating device may be part of the pressure measuring device it will typically be a separate entity such as a smartphone as discussed above. Preferably, the evaluating device may be a device which is portable and/or wearable by the user. Preferably, the evaluating device may be a smartphone, a tablet or a smartwatch.

Preferably, the elastic body may be substantially ball-shaped or roll-shaped.

Preferably, in case the elastic body is substantially ball-shaped, the elastic body may have an outer diameter ranging from 30 mm to 80 mm, preferably from 40 mm to 70 mm, most preferably from 50 mm to 60 mm.

Preferably, the energy source may be at least one, preferably rechargeable, lithium-ion battery.

Preferably, the pressure detection device may be configured to detect an absolute pressure range of 80 kPa to 200 kPa.

Preferably, the pressure detection device may have a detecting resolution in a range from 0.1 Pa to 20 Pa, preferably from 0.5 Pa to 15 Pa, more preferably from 0.7 Pa to 10 Pa, most preferably from 0.9 Pa to 5 Pa.

Preferably, the elastic body may be made of a synthetic or natural polymer material like silicone, polyethylene, polyurethane, soft PVC, (natural) rubber and/or latex, preferably of silicone.

Preferably, the elastic body may have a wall with a first section and a stiffened second section which is substantially stiffer than the first section. The first section and the stiffened second section may be made of the same material, e.g., the same polymers and/or elastomers. Alternatively, the first section and the stiffened second section may be made of different materials, e.g., different polymers and/or elastomers.

The stiffened second section may provide a housing-like section for the components, such as the pressure detection device, arranged in the pressure measuring device to be at least partially disposed in and/or attached to. This may prevent or at least limit damage to these components, for instance when the user compresses the elastic body and thus the lumen.

Preferably, the pressure detection device, and preferably also the energy source, may be arranged at least partially within the second section.

Further disclosed herein is a method for monitoring a medical condition of a user related to arthritis and/or arthrosis by means of a pressure measuring device according to any of the embodiments disclosed herein. The effects and advantages related to the pressure measuring device, as described above, apply to the method accordingly. The method is not covered by the claims.

The method comprises the step of providing an elastic body defining a lumen filled with a compressible fluid. The lumen is configured to be compressed by at least one body part of the user.

The method further comprises the step of compressing the elastic body by the body part of the user and thereby changing the absolute pressure of the compressible fluid within the lumen.

The elastic body may be compressed by multiple body parts of the user, simultaneously and/or sequentially. For instance, the elastic body may be compressed by at least portions of both hands of the user, simultaneously and/or sequentially.

Moreover, the method comprises the step of detecting at least one absolute pressure in the lumen and generating a pressure signal based on the detected absolute pressure by means of a pressure detection device arranged at least partially within the lumen.

Prior to detecting an absolute pressure in the lumen, the pressure detection device may be tared.

In addition, the method comprises the step of providing the pressure signal to an evaluating device.

Preferably, the detected absolute pressure may be compared with at least one predetermined upper absolute pressure value and/or at least one predetermined lower absolute pressure value and a warning signal may be triggered if the detected absolute pressure is less than the predetermined lower absolute pressure value and/or greater than the predetermined upper absolute pressure value.

Preferably, at least one temperature within the lumen may be detected by means of a temperature detection device arranged at least partially within the lumen.

For instance, it may be advantageous to cool the pressure measuring device prior to use thereof by the user, e.g., to alter properties of the pressure measuring device and/or to provide comforting effects to the user during use.

Thus, the temperature detection device may detect the temperature within the lumen during such a cooling process. Optionally, the temperature detection device may provide a signal or a warning to the user when the detected temperature reaches and/or falls below a predetermined temperature threshold.

The temperature detection device and the pressure detection device may be configured as a single, coherent unit. Alternatively, the temperature detection device and the pressure detection device may be configured as separate units.

Preferably, a tremor of the user may be detected by means of an accelerometer arranged at least partially within the pressure measuring device.

Preferably, the frequency and/or the amplitude of the detected tremor may be determined based on the tremor signal and a warning signal may be triggered if: the determined frequency surpasses a predetermined frequency and/or the determined amplitude surpasses a predetermined amplitude, and/or the determined frequency falls within a predetermined frequency range and/or the determined amplitude falls within a predetermined amplitude range.

Preferably, the pressure measuring device may vibrate by means of at least one vibration device arranged within the elastic body for at least one of the following events: upon the evaluating device being communicatively connected with at least the pressure detection device, and when the user compresses the lumen of the elastic body while the evaluating device is communicatively disconnected from the pressure detection device.

In the following, different possible compressing configurations, in which the user may compress the elastic body with different body parts and/or using different motions of certain body parts of the user and/or using different repetitions to achieve the effects and advantages of the present disclosure for different compressing configurations, are described.

Preferably, the elastic body is compressed by the body part of the user a multiple amount of times sequentially. Preferably, each compression of the elastic body is spaced from another compression of the elastic body by a maximum time period of 3 seconds, preferably 2.6 seconds, more preferably 2.2 seconds, more preferably 1.8 seconds, more preferably 1.4 seconds, more preferably 1 second, more preferably 0.8 seconds, most preferably 0.6 seconds.

Preferably, the elastic body is continuously compressed by the body part of the user for at least 0.4 seconds, preferably at least 0.8 seconds, more preferably at least 1.2 seconds, more preferably at least 1.6 seconds, more preferably at least 2 seconds, more preferably at least 2.5 seconds, more preferably at least 3 seconds, more preferably at least 3.5 seconds, most preferably at least 4 seconds.

The user may continuously compress the elastic body to a substantially constant degree or at varying degrees during the compression of the elastic body. For instance, the user may first compress the elastic body during a first time period to a higher degree and in a subsequent second period to a lower degree or vice versa. The user may also compress the elastic body with varying degrees of compression in an alternating manner.

Preferably, the elastic body is compressed by at least two fingers of the user, preferably between the fingers. Preferably, the two fingers are on one hand of the user or a first finger is on one hand and a second finger being on the other hand of the user.

Preferably, the elastic body is compressed by at least one palm and/or wrist of a hand of a user, preferably by the palms and/or wrists of both hands of the user, preferably between both palms and/or wrists.

Preferably, at least one joint of the body part or proximate the body part compressing the elastic body is flexed, preferably by at least 15°, more preferably by at least 30°, more preferably by at least 45°, about an axis of the joint while the elastic body is being compressed and/or prior to the elastic body being compressed.

In case the joint of the body part or proximate the body part compressing the elastic body is flexed prior to the elastic body being compressed, preferably the joint of the body part compressing the elastic body is flexed for less than 3 seconds, more preferably less than 2.5 seconds, more preferably less than 2 seconds, preferably less than 1.5 seconds, preferably less than 1 second, prior to the elastic body being compressed.

For instance, at least one joint or multiple joints in at least one finger, preferably multiple fingers, of at least one hand of the user may be flexed just before compressing the elastic body with the hand, or more specifically with the finger(s), of the user.

The elastic body may also be compressed with the palm of one hand or the palms of both hands while rotating the wrist of the hand or the wrists of both hands.

The object as set out at the beginning is also achieved by a kit with a computer-implemented evaluation program according to claim 7.

The effects and advantages related to the pressure measuring device, as described above, apply to the computer-implemented evaluation program accordingly.

Preferably, the evaluation program may be configured to provide information related to a medical condition of a user related to arthritis and/or arthrosis to the user.

The evaluation program may be configured to display a graphical user interface on a display of the evaluating device to display the information related to a medical condition of a user related to arthritis and/or arthrosis to the user.

The evaluation program may be configured to process, and preferably also store, data related to a medical condition of a user related to arthritis and/or arthrosis and provide various information to the user based on the generated pressure signal, such as at least one of a historical development over a specific time, warnings when the detected absolute pressure exceeds or falls below predetermined thresholds and suggested treatments including movement therapy, such as physical therapy and/or ergotherapy and/or drug treatment.

The evaluation program may be configured to provide the user with reminders, such as when pressure measurements and/or treatments should be performed.

Preferably, the evaluation program may be configured to compare the detected absolute pressure with at least one predetermined lower absolute pressure value and/or at least one predetermined upper absolute pressure value and trigger a warning signal if the detected absolute pressure is less than the predetermined lower absolute pressure value and/or greater than the predetermined upper absolute pressure value.

The warning signal may lead to a visual warning, preferably shown on a display of the evaluating device, and/or an acoustic warning.

Preferably, the evaluation program may be configured to generate a vibration triggering signal to activate at least one vibration device arranged at least partially within the pressure measuring device for at least one of the following events: upon the evaluating device being communicatively connected with at least the pressure detection device, and when the user compresses the lumen of the elastic body while the evaluating device is communicatively disconnected from the pressure detection device.

Preferably, the evaluation program may be configured to provide a warning to the user due to a tremor of the user if: the determined frequency of the tremor surpasses a predetermined frequency and/or the determined amplitude of the tremor surpasses a predetermined amplitude, and/or the determined frequency of the tremor falls within a predetermined frequency range and/or the determined amplitude of the tremor falls within a predetermined amplitude range.

The warning may be a visual warning, preferably shown on a display of the evaluating device and/or a light signal, and/or an acoustic warning and/or a warning by means of vibration of the device.

Preferred embodiments of the present invention are further elucidated below with reference to the figures. The described embodiments do not limit the present invention.
- Fig. 1: shows a sectional view of a kit according to an embodiment of the invention;
- Fig. 2: shows a perspective view of a kit according to a further embodiment of the invention;

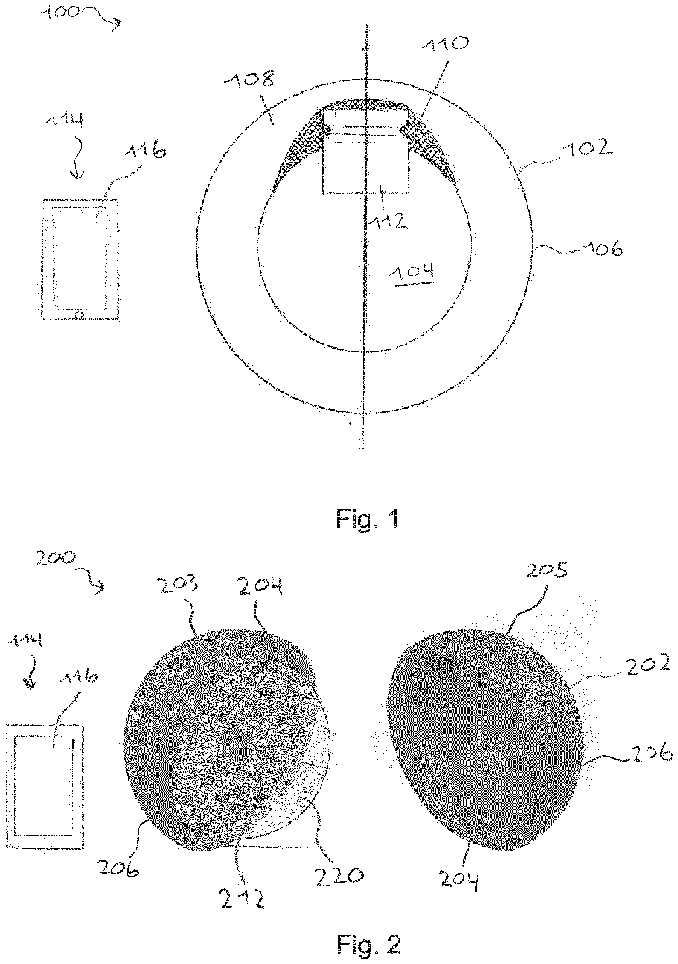

Fig. 1 shows a first embodiment of a pressure measuring device 100 for monitoring a medical condition of a user related to arthritis and/or arthrosis.

The pressure measuring device 100 comprises an elastic body 102 defining a lumen 104 filled with a compressible fluid, preferably air. Other types of compressible gases or liquids may also be used.

Alternatively or additionally, the lumen 104 of the elastic body 102 may at least partially be filled with a material whose physical state can be changed from a liquid to a solid and/or vice versa, preferably within a temperature range of -20 ° C and 150 ° C. Such a material may have a gel-like consistency in a first state and may be in a substantially solid state in a second state. Preferably, the material is in the first state at a first temperature and in the second state at a second temperature, the second temperature preferably being greater than the first temperature. Preferably, the first temperature is room temperature, preferably from 10° to 35°. Such a material may facilitate the manufacturing process of the pressure measuring device 100.

The lumen 104 is configured to be compressed by at least one body part, such as a hand, finger or a portion of a foot, of a user.

The lumen 104 is sealed from the environment surrounding the pressure measuring device 100 in a sufficiently fluid-tight manner.

The elastic body 102 may be a single integral body. Alternatively, the elastic body 102 may include two half-shells which are fixedly connected to each other, preferably in a sufficiently fluid-tight manner (see Fig. 2). Preferably, the two half-shells are adhesively connected to each other.

The elastic body 102 has a wall 106 with two different sections, a first section 108 and a second section 110. The second section 110 is stiffened such that the second section 110 is substantially stiffer than the first section 108. The second section 110 is integrated within a portion of the first section 108. The second section 110 may be stiffened by using a material which is stiffer than the material in the first section 108.

The pressure measuring device 100 further comprises an electronic unit 112 arranged partially within the second section 110 and partially within the lumen 104.

The electronic unit 112 may include a pressure detection device configured to detect an absolute pressure in the lumen 104, to generate a pressure signal based on the detected absolute pressure and to provide the pressure signal to an evaluating device 114 for evaluating the pressure signal.

The pressure measuring device 100 may be brought to a body part of a user, such as into a hand of the user.

The user may then apply a force to the pressure measuring device 100 via the body part, thereby compressing the elastic body 102 and the lumen 104.

Since the lumen 104 is sealed from the environment surrounding the pressure measuring device 100 in a sufficiently fluid-tight manner, the absolute pressure of the compressible fluid in the lumen 104 is increased substantially proportionally to the force applied to the pressure measuring device 100 by the user.

The pressure detection device of the electronic unit 112 detects the absolute pressure in the lumen 104 while the user is applying a force to the pressure measuring device 100 and compressing the elastic body 102 and the lumen 104.

The pressure detection device of the electronic unit 112 then generates a pressure signal based on the detected absolute pressure and provides the pressure signal to an evaluating device 114, for instance via a transmitting element, preferably via a wireless connection of the transmitting device to the evaluating device 114. The transmitting device may be included in the electronic unit 112.

The evaluating device 114 evaluates, i.e., processes, the pressure signal in order to provide information related to a medical condition of a user related to arthritis and/or arthrosis to the user.

The evaluating device 114 is configured as a computing device capable of processing and evaluating the pressure signal and preferably includes a display 116 for providing information related to conditions of the arthritis or arthrosis of the user based on the data detected by the pressure measuring device 100 via the display 116.

The information, such as warnings, may also be provided to the user visually, e.g., via text and/or lights, and/or acoustically, e.g., via sound and/or haptically, e.g., via vibration, via the evaluating device 114 and/or by a component arranged on or in the elastic body 102. The evaluating device 114 may be a third party component having a third party operating system.

The evaluating device 114 shown in Fig. 1 is configured as a smartphone. Alternatively, the evaluating device 114 may be configured as a smartwatch, smart glasses, tablet or any other device which is mobile, and preferably wearable by the user.

The evaluating device 114 may also be configured as a PC or any other stationary computing device capable of processing and evaluating the pressure signal.

The electronic unit 112 further includes an energy source arranged within the elastic body 102. The energy source is configured to provide electrical energy to at least the pressure detection device.

The electronic unit 112 may further include at least one vibration device arranged at least partially within the pressure measuring device 100. The vibration device is configured to vibrate upon receiving a vibration triggering signal.

The pressure measuring device 100 may be configured to generate the vibration triggering signal to activate the vibration device 112 for at least one of the following events: upon the evaluating device being communicatively connected with at least the pressure detection device 112, and when the user compresses the lumen 104 of the elastic body 102 while the evaluating device 114 is communicatively disconnected from the pressure detection device 112.

The electronic unit 112 may further include at least one accelerometer or gyroscope arranged at least partially within the pressure measuring device 100. The accelerometer or gyroscope 112 may be configured to detect a tremor of the user and generate a tremor signal and provide the tremor signal to the evaluating device 114.

The pressure measuring device 100, in particular the evaluating device 114, may be configured to determine the frequency and/or the amplitude of the detected tremor based on the tremor signal and trigger a warning signal if: the determined frequency surpasses a predetermined frequency and/or the determined amplitude surpasses a predetermined amplitude, and/or the determined frequency falls within a predetermined frequency range and/or the determined amplitude falls within a predetermined amplitude range.

The evaluating device 114 may include a computer-implemented evaluation program implemented therein and configured to process and evaluate the received data, e.g., the pressure signal, and provide information related to a medical condition of a user related to arthritis and/or arthrosis to the user, for instance visually via the display 116.

While the electronic unit 112 is shown as a single coherent unit which may include a pressure detection device, at least one energy source, at least one vibration device, at least one transmitting device and at least one accelerometer or gyroscope, as described above, some or all of the components, such as pressure detection device and/or energy source and/or vibration device and/or transmitting device and/or accelerometer or gyroscope, may be separate from each other and/or arranged at individual locations in the pressure measuring device 100.

Fig. 2 shows a second embodiment of a pressure measuring device 200 for monitoring a medical condition of a user related to arthritis and/or arthrosis.

Similar to the pressure measuring device 100 shown in Fig. 1, the pressure measuring device 200 shown in Fig. 2 comprises an elastic body 202 defining a lumen 204 filled with a compressible fluid, preferably air. Other types of gases or liquids may also be used.

Alternatively or additionally, the lumen 204 of the elastic body 202 may at least partially be filled with a material whose physical state can be changed from a liquid to a solid and/or vice versa, preferably within a temperature range of -20 ° C and 150 ° C. Such a material may have a gel-like or liquid consistency in a first state and may be in a substantially solid state in a second state. Preferably, the material is in the first state at a first temperature and in the second state at a second temperature, the second temperature preferably being greater than the first temperature. Preferably, the first temperature is room temperature, preferably from 10° to 35°. Such a material may facilitate the manufacturing process of the pressure measuring device 200.

The lumen 204 is configured to be compressed by at least one body part, such as a hand, finger or a portion of a foot, of a user.

The elastic body 202 includes two half-shells 203, 205 which may be fixedly connected to each other, preferably in a substantially fluid-tight manner. Preferably, the two half-shells may be adhesively connected to each other. Each half-shell of the elastic body 102 has a wall 206.

The pressure measuring device 200 also includes an electronic unit 212 which, like the electronic unit 112 of pressure measuring device 100 shown in Fig. 1, may include a pressure detection device, at least one energy source, at least one vibration device, at least one transmitting device and at least one accelerometer or gyroscope. The pressure detection device, energy source, vibration device, transmitting device and accelerometer may be configured as described with respect to the embodiment of Fig. 1.

Fig. 2 also shows an evaluating device 114, which may be configured as the evaluating device 114 shown in Fig. 1.

The pressure measuring device 200 shown in Fig. 2 differs mainly from the pressure measuring device 100 shown in Fig. 1 in that the wall 206 does not include a stiffened section for securing the electronic unit 212.

Instead, the pressure measuring device 200 includes a compressible material 220 arranged within and at least partially filling the lumen 204. The compressible material 220 may include a sponge material and/or a foam material and/or a gel and/or a wadding. The electronic unit 212, preferably including a pressure detection device, at least one energy source, at least one vibration device, at least one transmitting device and at least one accelerometer or gyroscope, is arranged within the compressible material 220, substantially centrally in the lumen 204, to secure the electronic unit 212 and its components in the pressure measuring device 200.

Moreover, the compressible material 220 may improve the effect of the vibrations generated by the vibration device.

For instance, the compressible material 220 may improve the haptic appeal of the vibrations generated by the vibration device to the user, e.g., by improving the distribution of the vibrations within the elastic body 202 and/or by altering characteristics of the vibrations, such as the amplitude of the vibrations.

## Claims

1. A kit for monitoring a medical condition of a user related to arthritis and/or arthrosis, the kit comprising a pressure measuring device (100; 200) and an evaluating device (114), the pressure measuring device (100; 200) comprising:
an elastic body (102; 202) defining a lumen (104; 204) filled with a compressible fluid, wherein the lumen (104; 204) is configured to be compressed by at least one body part of a user;
a pressure detection device (112; 212) arranged at least partially within the lumen (104; 204), the pressure detection device (112; 212) being configured to
detect at least one absolute pressure in the lumen (104; 204); generate a pressure signal based on the detected absolute pressure; and
provide the pressure signal to the evaluating device (114) for evaluating the pressure signal; and
an energy source (112; 212) arranged within the elastic body (102; 202), preferably at least partially within the lumen (104; 204), the energy source (112; 212) being configured to provide electrical energy to at least the pressure detection device (112; 212);
**characterised in that**
the pressure measuring device (100; 200) and/or the evaluating device (114) is configured to compare the detected absolute pressure with at least one predetermined lower absolute pressure value and to trigger a warning signal if the detected absolute pressure is less than the predetermined lower absolute pressure value.

2. The kit according to claim 1, further comprising a compressible sponge or foam material (220) arranged within and at least partially filling the lumen (204), wherein the pressure detection device (212), and preferably also the energy source (212), are arranged at least partially within the sponge or foam material (220) to secure the pressure detection device (212), and preferably also the energy source (212), in the pressure measuring device (200).

3. The kit according to any of the preceding claims, further comprising at least one vibration device (112; 212) arranged at least partially within the pressure measuring device (100; 200), wherein the vibration device (112; 212) is configured to vibrate upon receiving a vibration triggering signal.

4. The kit according to claim 3, wherein the pressure measuring device (100; 200) and/or the evaluating device (114) is configured to generate the vibration triggering signal to activate the vibration device (112; 212) for at least one of the following events:
upon the evaluating device being communicatively connected with at least the pressure detection device (112; 212);
when the user compresses the lumen (104; 204) of the elastic body (102; 202) while the evaluating device (114) is communicatively disconnected from the pressure detection device (112; 212).

5. The kit according to any of the preceding claims, further comprising at least one accelerometer or gyroscope (112; 212) arranged at least partially within the pressure measuring device (100; 200), wherein the accelerometer or gyroscope (112; 212) and/or the evaluating device (114) is configured to detect a tremor of the user.

6. The kit according to claim 5, wherein the pressure measuring device (100; 200) and/or the evaluating device (114) is configured to determine the frequency and/or the amplitude of the detected tremor based on the tremor signal and trigger a warning signal if:
the determined frequency surpasses a predetermined frequency and/or the determined amplitude surpasses a predetermined amplitude,
and/or
the determined frequency falls within a predetermined frequency range and/or
the determined amplitude falls within a predetermined amplitude range.

7. A kit according to any of claims 1 to 6 with a computer-implemented evaluation program implemented in the evaluating device (114) of the kit, wherein the evaluation program is configured to provide information related to a medical condition of a user related to arthritis and/or arthrosis to the user, and wherein the evaluation program is configured to compare the detected absolute pressure with at least one predetermined lower absolute pressure value and trigger a warning signal if the detected absolute pressure is less than the predetermined lower absolute pressure value.

8. The kit with evaluation program according to claim 7, wherein the evaluation program is configured to generate a vibration triggering signal to activate at least one vibration device (112; 212) arranged at least partially within the pressure measuring device (100; 200) for at least one of the following events:
upon the evaluating device (114) being communicatively connected with at least the pressure detection device (112; 212), and
when the user compresses the lumen (104; 204) of the elastic body (102; 202) while the evaluating device (114) is communicatively disconnected from the pressure detection device (112; 212).

9. The kit with evaluation program according to claim 7 or 8, wherein the evaluation program is configured to provide a warning to the user due to a tremor if:
the determined frequency of the tremor surpasses a predetermined frequency and/or the determined amplitude of the tremor surpasses a predetermined amplitude,
and/or
the determined frequency of the tremor falls within a predetermined frequency range and/or the determined amplitude of the tremor falls within a predetermined amplitude range.

## Patentansprüche

1. Kit zum Überwachen eines medizinischen Zustands eines Benutzers im Zusammenhang mit Arthritis und/oder Arthrose, wobei das Kit eine Druckmessvorrichtung (100; 200) und eine Auswertevorrichtung (114) aufweist, wobei die Druckmessvorrichtung (100; 200) aufweist:
einen elastischen Körper (102; 202), der ein mit einem komprimierbaren Fluid gefülltes Lumen (104; 204) definiert, wobei das Lumen (104; 204) dazu eingerichtet ist, durch mindestens einen Körperteil eines Benutzers zusammengedrückt zu werden;
eine Druckdetektionsvorrichtung (112; 212), die zumindest teilweise im Lumen (104; 204) angeordnet ist, wobei die Druckdetektionsvorrichtung (112; 212) dazu eingerichtet ist,
mindestens einen Absolutdruck im Lumen (104; 204) zu detektieren;
ein Drucksignal auf Basis des detektierten Absolutdrucks zu erzeugen; und
der Auswertevorrichtung (114) das Drucksignal zum Auswerten des Drucksignals bereitzustellen; und
eine Energiequelle (112; 212), die im elastischen Körper (102; 202), vorzugsweise zumindest teilweise im Lumen (104; 204), angeordnet ist, wobei die Energiequelle (112; 212) dazu eingerichtet ist, elektrische Energie mindestens der Druckdetektionsvorrichtung (112; 212) bereitzustellen;
**dadurch gekennzeichnet, dass**
die Druckmessvorrichtung (100; 200) und/oder die Auswertevorrichtung (114) dazu eingerichtet ist, den detektierten Absolutdruck mit mindestens einem vorbestimmten unteren Absolutdruckwert zu vergleichen und ein Warnsignal auszulösen, wenn der detektierte Absolutdruck kleiner ist als der vorbestimmte untere Absolutdruckwert.

2. Kit nach Anspruch 1, das ferner ein komprimierbares Schwamm- oder Schaummaterial (220) aufweist, das im Lumen (204) angeordnet ist und dasselbe zumindest teilweise füllt, wobei die Druckdetektionsvorrichtung (212), und vorzugsweise auch die Energiequelle (212), zumindest teilweise im Schwamm- oder Schaummaterial (220) angeordnet sind, um die Druckdetektionsvorrichtung (212) und vorzugsweise auch die Energiequelle (212) in der Druckmessvorrichtung (200) zu sichern.

3. Kit nach einem der vorstehenden Ansprüche, das ferner mindestens eine Vibrationsvorrichtung (112; 212) aufweist, die zumindest teilweise in der Druckmessvorrichtung (100; 200) angeordnet ist, wobei die Vibrationsvorrichtung (112; 212) dazu eingerichtet ist, bei Empfang eines Vibrationsauslösesignals zu vibrieren.

4. Kit nach Anspruch 3, wobei die Druckmessvorrichtung (100; 200) und/oder die Auswertevorrichtung (114) dazu eingerichtet ist, das Vibrationsauslösesignal zu erzeugen, um die Vibrationsvorrichtung (112; 212) bei mindestens einem der folgenden Ereignisse zu aktivieren:
beim kommunikativen Verbinden der Auswertevorrichtung mit mindestens der Druckdetektionsvorrichtung (112; 212); und
beim Zusammendrücken des Lumens (104; 204) des elastischen Körpers (102; 202) durch den Benutzer, während die Auswertevorrichtung (114) von der Druckdetektionsvorrichtung (112; 212) kommunikativ getrennt ist.

5. Kit nach einem der vorstehenden Ansprüche, das ferner mindestens einen Beschleunigungsmesser oder ein Gyroskop (112; 212) aufweist, der/das zumindest teilweise in der Druckmessvorrichtung (100; 200) angeordnet ist, wobei der Beschleunigungsmesser oder das Gyroskop (112; 212) und/oder die Auswertevorrichtung (114) dazu eingerichtet ist, einen Tremor des Benutzers zu detektieren.

6. Kit nach Anspruch 5, wobei die Druckmessvorrichtung (100; 200) und/oder die Auswertevorrichtung (114) dazu eingerichtet ist, die Frequenz und/oder die Amplitude des detektierten Tremors auf Basis des Tremorsignals zu ermitteln und ein Warnsignal auszulösen, wenn:
die ermittelte Frequenz eine vorbestimmte Frequenz überschreitet und/oder die ermittelte Amplitude eine vorbestimmte Amplitude überschreitet,
und/oder
die ermittelte Frequenz in einen vorbestimmten Frequenzbereich fällt und/oder die ermittelte Amplitude in einen vorbestimmten Amplitudenbereich fällt.

7. Kit nach einem der Ansprüche 1 bis 6 mit einem computerimplementierten Auswerteprogramm, das in der Auswertevorrichtung (114) des Kits implementiert ist, wobei das Auswerteprogramm dazu eingerichtet ist, dem Benutzer Informationen über einen medizinischen Zustand eines Benutzers im Zusammenhang mit Arthritis und/oder Arthrose bereitzustellen, und wobei das Auswerteprogramm dazu eingerichtet ist, den detektierten Absolutdruck mit mindestens einem vorbestimmten unteren Absolutdruckwert zu vergleichen und ein Warnsignal auszulösen, wenn der detektierte Absolutdruck kleiner ist als der vorbestimmte untere Absolutdruckwert.

8. Kit mit einem Auswerteprogramm nach Anspruch 7, wobei das Auswerteprogramm dazu eingerichtet ist, ein Vibrationsauslösesignal zu erzeugen, um mindestens eine Vibrationsvorrichtung (112; 212), die zumindest teilweise in der Druckmessvorrichtung (100; 200) angeordnet ist, bei mindestens einem der folgenden Ereignisse zu aktivieren:
beim kommunikativen Verbinden der Auswertevorrichtung (114) mit mindestens der Druckdetektionsvorrichtung (112; 212), und
beim Zusammendrücken des Lumens (104; 204) des elastischen Körpers (102; 202) durch den Benutzer, während die Auswertevorrichtung (114) von der Druckdetektionsvorrichtung (112; 212) kommunikativ getrennt ist.

9. Kit mit einem Auswerteprogramm nach Anspruch 7 oder 8, wobei das Auswerteprogramm dazu eingerichtet ist, den Benutzer infolge eines Tremors zu warnen, wenn:
die ermittelte Frequenz des Tremors eine vorbestimmte Frequenz überschreitet und/oder die ermittelte Amplitude des Tremors eine vorbestimmte Amplitude überschreitet,
und/oder
die ermittelte Frequenz des Tremors in einen vorbestimmten Frequenzbereich fällt und/oder die ermittelte Amplitude des Tremors in einen vorbestimmten Amplitudenbereich fällt.

## Revendications

1. Kit de surveillance d'un état médical d'un utilisateur en lien avec l'arthrite et/ou l'arthrose, le kit comprenant un dispositif de mesure de pression (100 ; 200) et un dispositif d'évaluation (114), le dispositif de mesure de pression (100 ; 200) comprenant :
un corps élastique (102 ; 202) définissant une lumière (104 ; 204) remplie d'un fluide compressible, dans lequel la lumière (104 ; 204) est configurée pour être comprimée par au moins une partie de corps d'un utilisateur ;
un dispositif de détection de pression (112 ; 212) agencé au moins partiellement à l'intérieur de la lumière (104 ; 204), le dispositif de détection de pression (112 ; 212) étant configuré pour
détecter au moins une pression absolue dans la lumière (104 ; 204) ;
générer un signal de pression sur la base de la pression absolue détectée ; et
fournir le signal de pression au dispositif d'évaluation (114) pour évaluer le signal de pression ; et
une source d'énergie (112 ; 212) agencée à l'intérieur du corps élastique (102 ; 202), de préférence au moins partiellement à l'intérieur de la lumière (104 ; 204), la source d'énergie (112 ; 212) étant configurée pour fournir de l'énergie électrique au moins au dispositif de détection de pression (112 ; 212) ;
**caractérisé en ce que**
le dispositif de mesure de pression (100 ; 200) et/ou le dispositif d'évaluation (114) est configuré pour comparer la pression absolue détectée à au moins une valeur de pression absolue inférieure prédéterminée et pour déclencher un signal d'alarme si la pression absolue détectée est inférieure à la valeur de pression absolue inférieure prédéterminée.

2. Kit selon la revendication 1, comprenant en outre un matériau spongieux ou en mousse compressible (220) agencé à l'intérieur de la lumière (204) et la remplissant au moins partiellement, dans lequel le dispositif de détection de pression (212), et de préférence également la source d'énergie (212), sont agencés au moins partiellement à l'intérieur du matériau spongieux ou en mousse (220) pour bloquer le dispositif de détection de pression (212), et de préférence également la source d'énergie (212), dans le dispositif de mesure de pression (200).

3. Kit selon l'une quelconque des revendications précédentes, comprenant en outre au moins un dispositif de vibration (112 ; 212) agencé au moins partiellement à l'intérieur du dispositif de mesure de pression (100 ; 200), dans lequel le dispositif de vibration (112 ; 212) est configuré pour vibrer lors de la réception d'un signal de déclenchement de vibration.

4. Kit selon la revendication 3, dans lequel le dispositif de mesure de pression (100 ; 200) et/ou le dispositif d'évaluation (114) est/sont configuré(s) pour générer le signal déclenchant la vibration pour activer le dispositif de vibration (112 ; 212) pour au moins l'un des événements suivants :
au moment où le dispositif d'évaluation est connecté communicativement avec au moins le dispositif de détection de pression (112 ; 212) ;
lorsque l'utilisateur comprime la lumière (104 ; 204) du corps élastique (102 ; 202) alors que le dispositif d'évaluation (114) est déconnecté communicativement du dispositif de détection de pression (112 ; 212).

5. Kit selon l'une quelconque des revendications précédentes, comprenant en outre au moins un accéléromètre ou gyroscope (112 ; 212) agencé au moins partiellement à l'intérieur du dispositif de mesure de pression (100 ; 200), dans lequel l'accéléromètre ou gyroscope (112 ; 212) et/ou le dispositif d'évaluation (114) est configuré pour détecter un tremblement de l'utilisateur.

6. Kit selon la revendication 5, dans lequel le dispositif de mesure de pression (100 ; 200) et/ou le dispositif d'évaluation (114) est configuré pour déterminer la fréquence et/ou l'amplitude du tremblement détecté sur la base du signal de tremblement et déclencher un signal d'alarme si :
la fréquence déterminée dépasse une fréquence prédéterminée et/ou si l'amplitude déterminée dépasse une amplitude prédéterminée,
et/ou
la fréquence déterminée se situe dans une plage de fréquences prédéterminée et/ou
l'amplitude déterminée se situe dans une plage d'amplitudes prédéterminée.

7. Kit selon l'une quelconque des revendications 1 à 6 avec un programme d'évaluation mis en oeuvre par ordinateur mis en oeuvre dans le dispositif d'évaluation (114) du kit, dans lequel le programme d'évaluation est configuré pour fournir des informations en lien avec un état médical d'un utilisateur en lien avec l'arthrite et/ou l'arthrose à l'utilisateur, et dans lequel le programme d'évaluation est configuré pour comparer la pression absolue détectée à au moins une valeur de pression absolue inférieure prédéterminée et pour déclencher un signal d'alarme si la pression absolue détectée est inférieure à la valeur de pression absolue inférieure prédéterminée.

8. Kit avec programme d'évaluation selon la revendication 7, dans lequel le programme d'évaluation est configuré pour générer un signal de déclenchement de vibration pour activer au moins un dispositif de vibration (112 ; 212) agencé au moins partiellement à l'intérieur du dispositif de mesure de pression (100 ; 200) pour au moins l'un des événements suivants :
au moment où le dispositif d'évaluation (114) est connecté communicativement avec au moins le dispositif de détection de pression (112 ; 212) ; et
lorsque l'utilisateur comprime la lumière (104 ; 204) du corps élastique (102 ; 202) alors que le dispositif d'évaluation (114) est déconnecté communicativement du dispositif de détection de pression (112 ; 212).

9. Kit avec programme d'évaluation selon la revendication 7 ou 8, dans lequel le programme d'évaluation est configuré pour fournir une alarme à l'utilisateur en raison d'un tremblement si :
la fréquence déterminée du tremblement dépasse une fréquence prédéterminée et/ou si l'amplitude déterminée du tremblement dépasse une amplitude prédéterminée,
et/ou
la fréquence déterminée du tremblement se situe dans une plage de fréquences prédéterminée et/ou si l'amplitude déterminée du tremblement se situe dans une plage d'amplitudes prédéterminée.
